# EUROPEAN PATENT SPECIFICATION

(11) **EP 2 346 441 B1**
(45) Date of publication and mention of the grant of the patent: **27.03.2013**
(21) Application number: 09760401.1
(22) Date of filing: 19.11.2009
(51) Int. Cl.: A61F 2/16

(54) **HARD AND SOFT TIP INTRAOCULAR LENS INJECTOR SYSTEM**
INJEKTIONSSYSTEM MIT HARTER UND WEICHER SPITZE FÜR INTRAOKULARLINSEN
SYSTÈME D'INJECTION DE CRISTALLIN ARTIFICIEL À POINTE DURE ET MOLLE

(30) Priority: 19.11.2008 US 273715
(43) Date of publication of application: 27.07.2011
(73) Proprietor: Bausch & Lomb Incorporated, Rochester, NY 14604-2701 (US)
(72) Inventor: WAGNER, Christopher, E., Webster NY 14580 (US)
(74) Representative: Vossius & Partner
(86) International application number: PCT/US2009/065115
(87) International publication number: WO 2010/059805

(56) References cited:
- EP-A1- 1 941 846
- WO-A1-2006/059183
- WO-A1-2007/080868

## Description

### Background of the Invention

The present invention relates to intraocular lens injector systems, and more particularly relates to an intraocular lens ("IOL") injector system having a rigid lens engagement surface for advancing the IOL within the injector body during a first stage of plunger advancement, and a soft lens engagement surface for advancing the IOL during a second stage of plunger advancement which concludes by expressing the IOL from the injector and into an eye.

IOLs are well known and are used for implanting into an eye to replace the eye's extracted natural lens in a common surgical procedure known as cataract surgery. There are many different types of IOLs available and the surgeon chooses the IOL according to one or more factors including, for example, the physiology and refractive needs of the patient's eye. IOLs are configured with an optic and one or more haptics extending from the optic which act as anchoring elements to properly position the IOL within the eye. The IOL is implanted in the eye with the optic aligned along the eye's visual axis. The IOL may be implanted in a variety of locations within the eye, but typically is positioned within the capsular bag from which the natural lens has been extracted.

An IOL is implanted in an eye with the aid of an implantation tool such as an IOL injector having a main body portion with a lumen and a plunger telescoping within the lumen. The IOL is placed inside the lumen and the injector tip is inserted into an incision made in the eye. The plunger is then advanced with the lens engagement surface of the plunger tip engaging and pushing the IOL out of the injector tip and into the eye. The injector tip tapers inwardly toward the tip opening causing the IOL to compress as it is pushed further therethrough by the injector tip. Since IOLs are very delicate, there is the chance the plunger tip may damage the IOL as it is pushed, compressed and ultimately ejected from the injector tip by the plunger. The highest (peak) delivery forces against the IOL occur at the injector tip opening, the diameter of which is typically less than about 3mm, for example.

Plunger tips are designed with a lens engagement surface for engaging and pushing the IOL through the lumen and out the injector tip opening. Both rigid and soft lens engagement surfaces have been proposed in the prior art with each having their own advantages and disadvantages. For example, a plunger tip having a lens engagement surface made of a rigid material has the benefit of providing a secure and controlled engagement profile with the IOL, particularly as the IOL is advanced within the injector lumen. However, a rigid lens engagement surface also has the disadvantage of possibly damaging the IOL due to the unyielding characteristic of the rigid material, particularly at the point of peak delivery force at the tip opening as described above. On the other hand, a plunger tip having a lens engagement surface made of a soft material has the benefit of yielding to the lens during the period of peak delivery force which reduces the risk of damaging the lens, but has the disadvantage of not providing as secure and controlled engagement profile with the IOL as does a rigid plunger tip lens engagement surface.

WO 2006/059183 A1 discloses a two-stage plunger for an IOL injector device including a shaft with a distal tip and a compressible sleeve positioned about the shaft with the tip extending forwardly of the sleeve, wherein in a first stage of IOL advancement through the injector device, the plunger tip engages and pushes the intraocular lens through a first section of the injector body, and in a second stage of IOL advancement, the sleeve enters the narrowing section of the injector body toward the open tip thereof with the IOL and sleeve both undergoing compression.

EP 1 941 846 A1 describes an IOL insertion device for inserting an IOL capable of securely and safely pushing out the lens by a simple structure, the IOL insertion device comprising: a main body having a lens placement portion on which the lens with a pair of loop parts at its optic part is placed, a transition portion deforming the lens, and a nozzle portion discharging the lens; and a lens push-out mechanism pushing out the lens placed on the lens placement portion, wherein the lens push-out mechanism comprises a plunger pushing out the lens and a slider having a lens contact part larger than the plunger.

There therefore remains a need for an improved IOL injector system and method for injecting an IOL into an eye which incorporates the benefits of both a rigid and soft plunger tip lens engagement surface while at the same time reduces or eliminates the disadvantages associated therewith.

### Summary of the Invention

The present invention as disclosed in the appended claims addresses the above-described need by providing an IOL injector system including a plunger assembly slidably disposed within the lumen of an injector body extending to an injector tip having an opening. The plunger assembly includes a first component comprising a rigid body having a lens engagement surface at its distal end and an interference feature. A compressible sleeve is slidably positioned on the first component. The plunger assembly further includes a second component having proximal and distal ends and a central opening extending longitudinally therebetween. The first component is slidably disposed within the central opening of the second component. The first component, the second component, and the sleeve are configured to slide within the lumen in a first state in which the engagement surface is disposed distally of the compressible sleeve and the IOL is carried in the lumen by the rigid lens engagement surface, thus having the benefit of a secure and control led lens engagement profile as described above.

The second component of the plunger assembly is configured such that, after the interference feature of the first component interferes with a portion of the injector body, the second component moves relative to the first component causing at least a portion of the sleeve to slide on the first component to become positioned distally of the lens engagement surface. Once the IOL approaches the injector tip, at least a portion of the compressible sleeve advances past the lens engagement surface of the first component and takes over pushing the IOL through the tip and out the tip opening. As mentioned above, the forces imparted on the IOL peak as the IOL is pushed through the tip opening. With the soft, compressible sleeve pushing the IOL out of the tip opening, the benefit of reducing the risk of damage to the IOL during peak delivery force is realized.

A method of injecting an intraocular lens into an eye (not part of the invention as claimed), comprises the steps of providing a plunger assembly and an injector body comprising a lumen extending to an injector tip, the plunger assembly disposed in the lumen, then sliding the plunger assembly within the lumen in a first suite during which a first component comprising a rigid lens engagement surface is in contact with the intraocular lens, then interfering the first component with the injector body, and then sliding a second component relative to the first component to move the sleeve relative to the first component to achieve a second state in which the compressible sleeve is in contact with the IOL and the IOL lens exits the injector tip.

### Brief Description of the Drawing

FIG. 1 is a perspective view of an IOL injector system in accordance with an embodiment of the invention;
FIG. 2 is a cross-sectional view of the system of FIG. 1 showing the plunger assembly thereof in the beginning of a first stage of plunger assembly advancement;
FIG. 3 is the view of FIG. 2 showing the plunger assembly thereof at the end of the first stage of plunger assembly advancement;
FIG. 4 is the view of FIG. 2 showing the plunger assembly thereof in the second, concluding stage of plunger assembly advancement
FIG. 5A is a plan view of an embodiment of the second component 28 as seen in the direction of arrow 5A in FIG. 4;
FIG. 5B is a plan view of an embodiment of first component 22 as seen in the direction of arrow 5A in FIG. 4;
FIG. 6 is a fragmented, longitudinal cross-sectional view of another embodiment of the interface between the first and second components;
FIG. 7 is an enlarged, fragmented, perspective view of another embodiment of the injector body and interface between the first and second components; and
FIG. 7A is a fragmented, cross-sectional view thereof as taken along the line 7A-7A in Fig. 7.

### Detailed Description

An IOL is implanted in an eye by a surgeon with the aid of an injector tool such as IOL injector system 10. Injector system 10 includes an injector body 12 and a plunger assembly 14 which is slidably disposed within a lumen 16 of injector body 12. Lumen 16 of injector body 12 extends to an injector tip 18 terminating in an opening 18' wherethrough an IOL 20 may pass and thereby expressed from injector system 10 and into an eye (not shown).

Plunger assembly 14 includes a first component 22 comprising a shaft having a lens engagement surface 24 at distal end 22a thereof. First component 22 including lens engagement surface 24 is made from any suitable rigid material such as a metal or plastic, for example. An interference feature 26 is provided at proximal end 22b thereof for the purpose to be explained below.

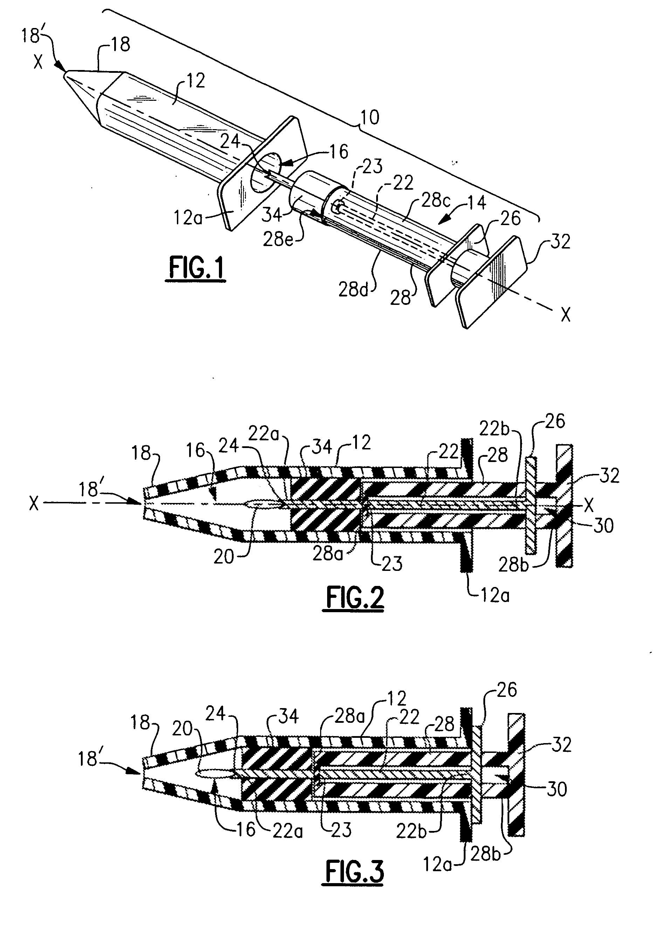

Plunger assembly 14 further includes a second component 28 having a central opening 30 extending longitudinally between distal and proximal ends 28a, 28b, respectively, and a thumb press 32 located at proximal end 28b. First component 22 is slidably disposed within central opening 30 of second component 28 and first component 22 are together slidably disposed within lumen 16 generally along longitudinal axis X-X seen in FIG. 1. Plunger assembly 14 is advanced within injector body 12 in the manner of a syringe by a user pressing their thumb against thumb press 32 with injector body 12 held between the fingers and against the distal facing surface of flange 12a.

As seen in FIG. 2, a compressible sleeve 34 is slidably positioned (e.g., via a press fit) on first component 22 in a first state with lens engagement surface 24 located distally of sleeve 34. Compressible sleeve 34 may be made of any suitable soft material which will compress such as a silicone elastomer, for example. Second component distal end 28a is located close to and abuts compressible sleeve 34. In this first state, thumb press 32 of second component 28 is spaced from interference feature 26 of first component 22.

FIGS. 2 to 3 show the first stage of plunger assembly advancement where, in FIG. 2, interference feature 26 is spaced from injector body 12. As a user presses upon thumb press 32 as described above, plunger assembly 14 slides within lumen 16 toward tip 18. A stop 23 may be provided on first component 22 to prevent sleeve 34 from sliding on first component 22 toward proximal end 22b thereof during advancement of plunger assembly 14 within lumen 16. FIG. 3 shows the conclusion of the first stage of plunger assembly advancement wherein interference feature 26 of first component 22 is abutting injector body 12 which prevents further advancement of first component 22 within injector body 12. This concludes the first stage of plunger assembly advancement wherein the lens engagement surface 24 has carried IOL 20 within lumen 16 toward tip 18 and is still located distally of sleeve 34.

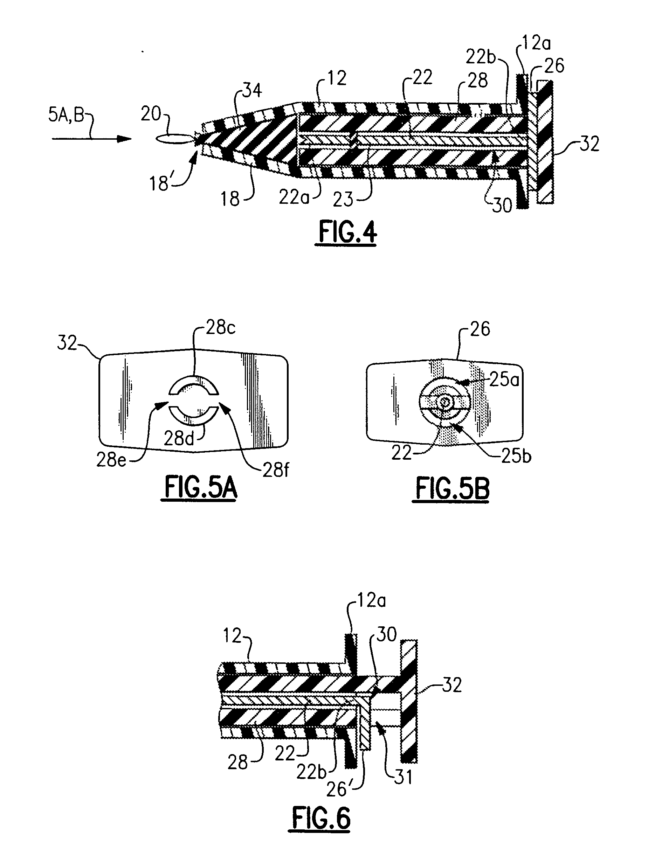

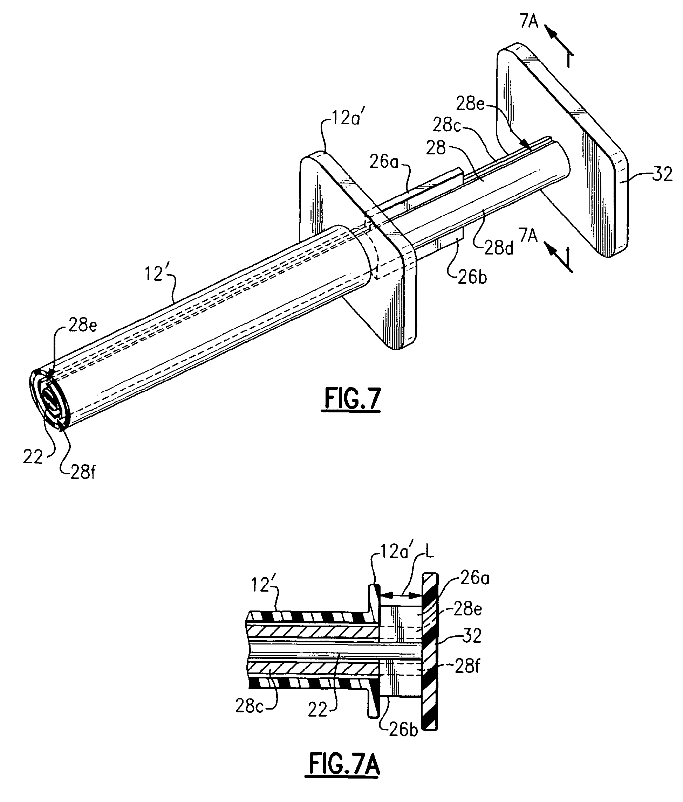

A variety of configurations may provide the interface between interference feature 26 and second component 28. In FIGS. 5A and B, second component 28 is bifurcated into two portions 28a and 28b which extend through first and second openings 25a and 25b, respectively, formed in interference feature 26. Although thumb press 32 and interference feature 26 are shown as generally rectangular in FIGS. 5A and B, other shapes are of course possible (e.g., square, circular, oval, etc.). In FIG. 6, second component 28 includes a longitudinally extending slot 31 wherethrough interference feature 26' extends. In FIGS. 7 and 7A, second component 28 is bifurcated into portions 28c and 28d as in the embodiment of FIG. 5A and interference feature 26 is formed as first and second flanges 16a and 26b which are adapted to extend through the longitudinally extending openings 28e and 28f defined between portions 28c and 28d, respectively. The length "L" of flanges 26a, 26b may vary to set the appropriate stopping point for second component 28 relative to body flange 12a'. In the embodiment of FIGS. 7, 7A, lens engagement surface 24, sleeve 34 and tip 18 are not shown for the sake of clarity. Also, body 12' is shown as circular in cross-section and interference feature 26a,b extends in a plane parallel to injector axis X-X while in the embodiments of FIGS. 1-6, body 12 is rectangular and interference feature 26 extends in a plane perpendicular to injector axis X-X. Other configurations for the injector parts including the injector body and the sliding interface between first and second components 22 and 28 allowing interference feature 26, 26', 26a,b to interfere with injector body 12 are of course possible as will be appreciated by those skilled in the art.

Once interference feature 26, 26' is slid into abutting contact with injector body 12, the user continues pressing on thumb press 32 causing second component 28 to slide in the distal direction relative to the first component 22 which itself is prevented from further advancement due to interference feature 26, 26' abutting injector body 12. During the second stage of plunger assembly advancement occurring between FIGS. 3 and 4, the space between interference feature 26 and thumb press 32 closes and second component distal end 28a pushes against sleeve 34 causing sleeve 34 to slide in the distal direction (toward injector tip 18) with respect to first component 22. Once the distal-most portion of sleeve 34 slides past lens engagement surface 24, it makes contact with and disengages IOL 20 from lens engagement surface 24 and begins advancing IOL 20 toward injector tip opening 18'. As sleeve 34 is pushed through injector tip 18, the sleeve compresses due to the inward tapering of the tip 18. Upon full advancement of second component 28, sleeve 34 expels IOL 20 from opening 18' as seen in FIG. 4.

It will be appreciated that during the first stage of plunger assembly advancement occurring between FIGS. 2 and 3, IOL 20 is carried through lumen 16 by lens engagement surface 24 which is rigid and thus has the benefit of a secure and controlled lens engagement profile as described above. Once interference feature 26, 26' abuts injector body 12 and second component 28 is advanced further with respect to the now stationary first component 22, at least a portion of compressible sleeve 34 is caused to advance past lens engagement surface 24 and thereby takes over pushing lens 20 through tip 18 and out opening 18'. As mentioned above, the forces imparted to IOL 20 peak as the IOL 20 is pushed through opening 18'. With soft sleeve 34 pushing the IOL 20 out of opening 18', the benefit of reducing the risk of damage to IOL 20 during peak forces imparted on IOL 20 is also realized.

There is thus provided an IOL injector system which obtains the benefit of a rigid lens engagement surface during a first stage of plunger assembly advancement, and the benefit of a soft lens engagement surface during the second or final stage of plunger assembly advancement during peak IOL delivery force.

## Claims

1. An intraocular lens injector system comprising:
A) a plunger assembly (14), comprising:
a) a first component (22) comprising a rigid body having a lens engagement surface (24) at
its distal end (22a) and an interference feature (26),
b) a compressible sleeve (34) positioned on said first component (22),
c) a second component (28) having proximal and distal ends (28a, 28b) and a a central opening (30) extending longitudinally therebetween, said first component (22) slidably disposed within said central opening, (30) and
B) an injector body (12) comprising a lumen (16) extending to an injector tip (18), the plunger assembly (14) being slidably disposed in the lumen (16),
the first component (22) and the sleeve (34) being configured to slide within the lumen (16) in a first state in which the engagement surface (24) is disposed distally of the compressible sleeve (34), and the second component (28) being configured such that, after the interference feature (26) interferes with a portion of the injector body (12), the second component (28) is adapted to move relative to the first component (22) so as to cause the sleeve (34) to be positioned distally of the engagement surface (24).

2. The intraocular lens injector system of claim 1, and further comprising a stop (23) positioned on said first component (22), said sleeve (34) positioned distally of said stop (23) and said stop (23) preventing said sleeve (34) from sliding proximally on said first component (22) past said stop (23).

3. The intraocular lens injector system of claim 1, wherein said second component (28) includes a longitudinally extending slot (31) wherethrough said interference feature (26) slidably extends.

4. The intraocular lens system of claim 1 wherein said second component (28) includes first and second portions (28a, 28b) adapted to slidably extend through first and second openings (25a, 25b) formed in said interference feature (26), respectively.

5. The intraocular lens system of claim 1 wherein said second component (28) includes first and second portions (28c, 28d) defining first and second longitudinally extending openings (28e, 28f) therebetween, and said interference feature (26) includes first and second flanges (26a, 26b) adapted to slidably extend through said first and second longitudinal openings (28e, 28f), respectively.

6. The intraocular lens system of claim 1 wherein said sleeve (34) is formed from a silicone elastomer.

7. The intraocular lens system of claim 1 wherein said injector body (12) includes a flange (12a) configured to interfere with said interference feature (26).

8. The intraocular lens system of claim 1 and further comprising a thumb press (32) disposed at said second component proximal end (28b).

## Patentansprüche

1. Intraokularlinseninjektorsystem mit:
A) einer Kolbenanordnung (14), die aufweist:
a) eine erste Komponente (22), die einen starren Körper mit einer Linseneingriffsfläche (24) an seinem distalen Ende (22a) und ein Zwischenelement (26) aufweist;
b) eine zusammendrückbare Hülse (34), die an der ersten Komponente (22) angeordnet ist;
c) eine zweite Komponente (28) mit einem proximalen und einem distalen Ende (28a, 28b) und einer zentralen Öffnung (30), die sich longitudinal dazwischen erstreckt, wobei die erste Komponente (22) verschiebbar in der zentralen Öffnung (30) angeordnet ist; und
B) einem Injektorkörper (12), der ein Lumen (16) aufweist, das sich zu einer Injektorspitze (18) erstreckt, wobei die Kolbenanordnung (14) verschiebbar im Lumen (16) angeordnet ist;
wobei die erste Komponente (22) und die Hülse (34) konfiguriert sind, in einem ersten Zustand, in dem sich die Eingriffsfläche (24) distal von der zusammendrückbaren Hülse (34) befindet, im Lumen (16) zu gleiten, und die zweite Komponente (28) so konfiguriert ist, dass, nachdem das Zwischenelement (26) auf einen Abschnitt des Injektorkörpers (12) einwirkt, die zweite Komponente (28) eingerichtet ist, sich relativ zur ersten Komponente (22) zu bewegen, um zu bewirken, dass die Hülse (34) distal von der Eingriffsfläche (24) angeordnet wird.

2. Intraokularlinseninjektorsystem nach Anspruch 1, das ferner einen Anschlag (23) aufweist, der an der ersten Komponente (22) angeordnet ist, wobei die Hülse (34) distal vom Anschlag (23) angeordnet ist und der Anschlag (23) verhindert, dass die Hülse (34) proximal auf der ersten Komponente (22) am Anschlag (23) vorbei gleitet.

3. Intraokularlinseninjektorsystem nach Anspruch 1, wobei die zweite Komponente (28) einen sich longitudinal erstreckenden Schlitz (31) aufweist, durch den sich das Zwischenelement (26) verschiebbar erstreckt.

4. Intraokularlinsensystem nach Anspruch 1, wobei die zweite Komponente (28) einen ersten und einen zweiten Abschnitt (28a, 28b) aufweist, die eingerichtet sind, sich verschiebbar durch eine erste bzw. eine zweite Öffnung (25a, 25b) zu erstrecken, die im Zwischenelement (26) ausgebildet sind.

5. Intraokularlinsensystem nach Anspruch 1, wobei die zweite Komponente (28) einen ersten und einen zweiten Abschnitt (28c, 28d) aufweist, die eine erste und eine zweite sich longitudinal erstreckende Öffnung (28e, 28f) dazwischen definieren, und das Zwischenelement (26) einen ersten und einen zweiten Flansch (26a, 26b) aufweist, die eingerichtet sind, sich verschiebbar durch die erste bzw. zweite longitudinale Öffnung (28e, 28f) zu erstrecken.

6. Intraokularlinsensystem nach Anspruch 1, wobei die Hülse (34) aus einem Silikonelastomer ausgebildet ist.

7. Intraokularlinsensystem nach Anspruch 1, wobei der Injektorkörper (12) einen Flansch (12a) aufweist, der konfiguriert ist, auf das Zwischenelement (26) einzuwirken.

8. Intraokularlinsensystem nach Anspruch 1, das ferner eine Daumenpresse (32) aufweist, die am proximalen Ende (28b) der zweiten Komponente angeordnet ist.

## Revendications

1. Système d'injecteur de lentille intraoculaire, comprenant :
A) un ensemble de plongeur (14), comprenant :
a) un premier composant (22), comprenant un corps rigide ayant une surface d'enclenchement de lentille (24) à
son extrémité distale (22a) et un élément d'interférence (26),
b) un manchon (34) compressible positionné sur ledit premier composant (22),
c) un deuxième composant (28) ayant des extrémités proximale et distale (28a, 28b) et une ouverture centrale (30) s'étendant longitudinalement en position intermédiaire, ledit premier composant (22) étant disposé de façon coulissante à l'intérieur de ladite ouverture centrale (30), et
B) un corps d'injecteur (12) comprenant une lumière (16) s'étendant jusqu'à un bout d'injecteur (18), l'ensemble de plongeur (14) étant disposé de façon coulissante dans la lumière (16),
le premier composant (22) et le manchon (34) étant configurés pour coulisser à l'intérieur de la lumière (16) dans un premier état dans lequel la surface d'enclenchement (24) est disposée de façon distale par rapport au manchon (34) compressible, et le deuxième composant (28) étant configuré de telle sorte que, après que l'élément d'interférence (26) a interféré avec une portion du corps d'injecteur (12), le deuxième composant (28) est adapté pour se déplacer par rapport au premier composant (22) de façon à amener le manchon (34) à être positionné de façon distale par rapport à la surface d'enclenchement (24).

2. Système d'injecteur de lentille intraoculaire selon la revendication 1, comprenant également une butée (23) positionnée sur ledit premier composant (22), ledit manchon (34) étant positionné de façon distale par rapport à ladite butée (23), et ladite butée (23) empêchant ledit manchon (34) de coulisser de façon proximale sur ledit premier composant (22) au-delà de ladite butée (23).

3. Système d'injecteur de lentille intraoculaire selon la revendication 1, dans lequel ledit deuxième composant (28) inclut une rainure (31) s'étendant longitudinalement et à travers laquelle s'étend de façon coulissante ledit élément d'interférence (26).

4. Système d'injecteur de lentille intraoculaire selon la revendication 1, dans lequel ledit deuxième composant (28) inclut des première et deuxième portions (28a, 28b) adaptées pour s'étendre de façon coulissante à travers les première et deuxième ouvertures (25a, 25b) formées respectivement dans ledit élément d'interférence (26).

5. Système d'injecteur de lentille intraoculaire selon la revendication 1, dans lequel ledit deuxième composant (28) inclut des première et deuxième portions (28c, 28d) définissant des première et deuxième ouvertures (28e, 28f) s'étendant longitudinalement en position intermédiaire, et ledit élément d'interférence (26) inclut des première et deuxième brides (26a, 26b) adaptées pour s'étendre de façon coulissante à travers lesdites première et deuxième ouvertures longitudinales (28e, 28f) respectivement.

6. Système d'injecteur de lentille intraoculaire selon la revendication 1, dans lequel ledit manchon (34) est formé en élastomère de silicone.

7. Système d'injecteur de lentille intraoculaire selon la revendication 1, dans lequel ledit corps d'injecteur (12) inclut une bride (12a) configurée pour interférer avec ledit élément d'interférence (26).

8. Système d'injecteur de lentille intraoculaire selon la revendication 1, comprenant également un poussoir (32) disposé au niveau de ladite extrémité proximale (28b) du deuxième composant.
